# EUROPEAN PATENT APPLICATION

(11) **EP 4 282 397 A1**
(43) Date of publication of application: **29.11.2023**
(21) Application number: 22775292.0
(22) Date of filing: 15.03.2022
(51) Int. Cl.: A61K 8/25, A61K 8/89, A61Q 19/00

(54) **COSMETIC PREPARATION**

(30) Priority: 26.03.2021 JP 2021053953
(71) Applicant: Shiseido Company, Ltd., Chuo-ku Tokyo 104-0061 (JP)
(72) Inventor: SUGIHARA, Megumi, Tokyo 104-0061 (JP); NISHIDA, Keita, Tokyo 104-0061 (JP)
(74) Representative: Cabinet Beau de Loménie
(86) International application number: PCT/JP2022/011702
(87) International publication number: WO 2022/202503

(57) **Abstract**

Provided is a cosmetic which contains a hydrophobized silica powder dispersed in an aqueous solvent and which is capable of absorbing sebum without whitening on the skin after application.

The cosmetic contains an aqueous solvent, a hydrophobized silica powder dispersed in the aqueous solvent, a non-volatile oil, and a dispersant, wherein (i) the hydrophobized silica powder has been subjected to a hydrophobic treatment with a silicone-based hydrophobizing agent and the non-volatile oil is a non-volatile silicone oil, or (ii) the hydrophobized silica powder has been subjected to a hydrophobic treatment with a fatty acid-based hydrophobizing agent and the non-volatile oil is a non-volatile hydrocarbon oil.

## Description

### FIELD

The present invention relates to a cosmetic comprising a hydrophobized silica powder which is dispersed in an aqueous solvent.

### BACKGROUND

In recent years, various cosmetics have been developed in accordance with need.

For example, Patent Literature 1 discloses, as a composition having a pore correction effect with a sensation similar to skin care cosmetics, a composition comprising an aqueous medium, a lipophilic porous powder which is dispersed in the aqueous medium, an oil having a viscosity of 100 mPa·s or less retained in at least part of the pores of the lipophilic porous powder, and a dispersant which disperses the lipophilic porous powder in the aqueous medium.

### [CITATION LIST]

### [PATENT LITERATURE]

[PTL 1] WO 2017/073758

### SUMMARY

### [TECHNICAL PROBLEM]

However, until now, regarding cosmetics comprising an aqueous solvent as a continuous phase, cosmetics comprising a hydrophobized silica powder as a lipophilic porous powder blended in an aqueous solvent have not been sufficiently studied.

Thus, in order to obtain a long-lasting makeup effect, the present inventors have worked to develop the blending of a hydrophobized silica powder as a lipophilic porous powder in an aqueous solvent. However, when applied to the skin, a cosmetic containing a hydrophobized silica powder blended in an aqueous solvent can absorbs sebum, but causes a problem that the hydrophobized silica powder results in a white appearance on the skin.

The problem that the blended powder looks unnaturally white on the skin after application is known, for example, in cosmetics blended with lipophilic porous powder, as in Patent Literature 1. Patent Literature 1 reports that simply adding crosslinked polymethyl methacrylate as a lipophilic porous powder to an aqueous dispersion medium results in whitening due to an excessive light scattering effect. In order to solve this problem, at least part of the pores of the crosslinked polymethyl methacrylate as the lipophilic porous powder retain an oil component having a viscosity of 100 mPa·s or less to appropriately suppress light scattering.

However, when a hydrophobized silica powder is used as the lipophilic porous powder, even if the same oil content as in Patent Literature 1 is retained in part of the pores of the hydrophobized silica powder, the problem of whitening on the skin could not be resolved.

The present invention is intended to improve the above circumstances, and an object thereof is to provide a cosmetic which comprises a hydrophobized silica powder dispersed in an aqueous solvent and which is capable of absorbing sebum without whitening on the skin after application.

The present invention, which achieves the object described above, is as described below.
<Aspect 1> A cosmetic, comprising:
   an aqueous solvent,
   a hydrophobized silica powder dispersed in the aqueous solvent,
   a non-volatile oil, and
   a dispersant, wherein
      (i) the hydrophobized silica powder has been subjected to a hydrophobic treatment with a silicone-based hydrophobizing agent and the non-volatile oil is a non-volatile silicone oil, or
      (ii) the hydrophobized silica powder has been subjected to a hydrophobic treatment with a fatty acid-based hydrophobizing agent and the non-volatile oil is a non-volatile hydrocarbon oil.
<Aspect 2> The cosmetic according to Aspect 1, wherein the hydrophobized silica powder has been subjected to a hydrophobic treatment with a dimethicone-based hydrophobizing agent and the non-volatile oil is a non-volatile dimethicone.
<Aspect 3> The cosmetic according to Aspect 1 or 2, wherein an oleic acid oil absorption amount of the hydrophobized silica powder is 1.0 ml/g or more.
<Aspect 4> The cosmetic according to any one of Aspects 1 to 3, wherein a content of the hydrophobized silica powder is 1.0 to 20 mass%.
<Aspect 5> The cosmetic according to any one of Aspects 1 to 4, wherein a content of the non-volatile oil is 1.0 to 40 mass%.
<Aspect 6> The cosmetic according to any one of Aspects 1 to 5, wherein the cosmetic is for use in pore correction.
<Aspect 7> The cosmetic according to any one of Aspects 1 to 6, wherein a viscosity of the cosmetic is 100000 mPa.s or less at 25°C.
<Aspect 8> The cosmetic according to any one of Aspects 1 to 7, wherein a ratio of a volumetric content of the non-volatile oil to a maximum oil absorption amount of the hydrophobic silica powder ("volumetric content of non-volatile oil" / "maximum oil absorption amount of hydrophobic silica powder") is 0.3 or greater and less than 1.0.

### [ADVANTAGEOUS EFFECTS OF INVENTION]

According to the present invention, there can be provided a cosmetic which comprises a hydrophobized silica powder dispersed in an aqueous solvent and which is capable of absorbing sebum without whitening on the skin after application.

### <<Cosmetic>>

The cosmetic of the present invention comprises:
an aqueous solvent,
a hydrophobized silica powder dispersed in the aqueous solvent,
a non-volatile oil, and
a dispersant, wherein
   (i) the hydrophobized silica powder has been subjected to a hydrophobic treatment with a silicone-based hydrophobizing agent and the non-volatile oil is a non-volatile silicone oil, or
   (ii) the hydrophobized silica powder has been subjected to a hydrophobic treatment with a fatty acid-based hydrophobizing agent and the non-volatile oil is a non-volatile hydrocarbon oil.

As a result of intensive research by the present inventors on the problem that a hydrophobized silica powder turns white on the skin after application, it was found that this problem could be solved by selecting an appropriate combination of a hydrophobized silica powder and a non-volatile oil, leading to the completion of the present invention.

The appropriate combination of a hydrophobized silica powder and a non-volatile oil is either (i) a combination wherein the hydrophobized silica powder has been subjected to a hydrophobic treatment with a silicone-based hydrophobizing agent and the non-volatile oil is a non-volatile silicone oil, or (ii) a combination wherein the hydrophobized silica powder has been subjected to a hydrophobic treatment with a fatty acid-based hydrophobizing agent and the non-volatile oil is a non-volatile hydrocarbon oil.

Furthermore, specific examples of appropriate combinations include, but are not limited to, (i-1) a combination wherein the hydrophobized silica powder has been subjected to a hydrophobic treatment with a dimethicone-based hydrophobizing agent and the non-volatile oil is a non-volatile dimethicone; and (ii-1) a combination wherein the hydrophobized silica powder is subjected to a hydrophobic treatment with a fatty acid-based hydrophobizing agent and the non-volatile oil is a hydrogenated polydecene.

### <Hydrophobized Silica Powder>

The cosmetic of the present invention comprises a hydrophobized silica powder dispersed in an aqueous solvent. By containing the hydrophobized silica powder, sebum can be absorbed, and thus, makeup deterioration due to sebum can be prevented. Furthermore, the hydrophobized silica powder penetrates into the recesses of the pores of the skin, providing the effect of making the unevenness of the pores visually inconspicuous.

In the present invention, the term "hydrophobized silica powder" refers to a silica powder having a surface which has been subjected to hydrophobic treatment. The hydrophobic treatment may be a treatment with a silicone-based hydrophobizing agent or a treatment with a fatty acid-based hydrophobizing agent.

More specifically, examples of silicone-based hydrophobizing agents include, but are not limited to, dimethicone-based hydrophobizing agents and methicone-based hydrophobizing agents. Examples of fatty acid-based hydrophobizing agents include, but are not limited to, stearic acid-based hydrophobic treatment agents and isostearic acid-based hydrophobic treatment agents.

In the present invention, the silica powder may be porous silica particles or silica particles having uneven surfaces, but from the viewpoint of improving the sebum absorption effect, porous silica particles are preferable. The shapes of the silica particles are not particularly limited, and may be, for example, spherical, substantially spherical, or spheroidal.

The average particle size of the hydrophobized silica powder is not particularly limited, and may be, for example, 2.0 µm or more, 3.0 µm or more, 4.0 µm or more, or 5.0 µm or more, and may be 20 µm or less, 15 µm or less, or 10 µm or less. In the present invention, the average particle size can be calculated as the number average of the equivalent diameters of projected circles of the primary particles observed by SEM or TEM.

In the present invention, the oil absorption amount of the hydrophobized silica powder is evaluated by the amount (ml) of oleic acid which can be absorbed per gram of hydrophobized silica powder (i.e., "oleic acid oil absorption amount (ml/g)"). In the present invention, from the viewpoint of improving the sebum absorption effect, the oleic acid oil absorption amount of the hydrophobized silica powder may be 1.0 ml/g or more, 1.1 ml/g or more, 1.2 ml/g or more, 1.3 ml/g or more, 1.4 ml/g or more, 1.5 ml/g or more, 1.6 ml/g or more, 1.7 ml/g or more, 1.8 ml/g or more, 1.9 ml/g or more, 2.0 ml/g or more, 2.1 ml/g or more, 2.2 ml/g or more, or 2.3 ml/g or more, and from the viewpoint of uniformly absorbing sebum on the skin, it may be 8.0 ml/g or less.

Note that the oleic acid oil absorption amount of the hydrophobized silica powder can be determined with reference to "JIS-K 5101-13-1, Japan Industrial Standards, Pigment test methods - Part 13: Oil absorption amount - Section 1: Refined linseed oil method."

In the present invention, a commercially available product may be used as the hydrophobized silica powder.

In the cosmetic of the present invention, from the viewpoint of increasing the sebum absorption effect, the content of the hydrophobized silica powder, relative to the total amount of the cosmetic, may be 1.0 mass% or more, 2.0 mass% or more, 3.0 mass% or more, 4.0 mass% or more, 5.0 mass% or more, 6.0 mass% or more, 7.0 mass% or more, 8.0 mass% or more, 9.0 mass% or more, 10 mass% or more, 11 mass% or more, 12 mass% or more, 13 mass% or more, 14 mass% or more, 15 mass% or more, 16 mass% or more, 17 mass% or more, or 18 mass% or more, and from the viewpoint of dispersibility, may be, relative to the total amount of the cosmetic, 30 mass% or less, 29 mass% or less, 28 mass% or less, 27 mass% or less, 26 mass% or less, 25 mass% or less, 24 mass% or less, 23 mass% or less, 22 mass% or less, or 21 mass% or less.

In the cosmetic of the present invention, the hydrophobized silica powder is dispersed in an aqueous solvent. In a preferable dispersed state of hydrophobized silica powder, for example, the sedimentation rate is 1.0 mass% or less when allowed to stand at 25°C for one day. Measurement of the sedimentation rate may be performed with reference to, for example, the "soil particle size test method" specified in JIS A 1204:2020.

### <Non-Volatile Oil>

The cosmetic of the present invention comprises a non-volatile oil. The non-volatile oil may be a non-volatile silicone oil or a non-volatile hydrocarbon oil. In the present invention, "non-volatile" is intended for a volatile matter content of 5% or less when allowed to stand at 105°C for 3 hours. The volatile matter content refers to the value of the weight change rate measured by the gravimetric method after placing a filter paper on a glass petri dish and dropping approximately 0.2 g of a sample thereon. In contrast, "volatile" is intended for a volatile matter content of greater than 5% when allowed to stand at 105°C for 3 hours at atmospheric pressure.

In the present invention, examples of non-volatile silicone oil include non-volatile dimethicone, caprylyl methicone, and phenyl-modified silicone. Non-volatile dimethicone is most preferable.

In the present invention, examples of the non-volatile hydrocarbon oil include, but are not limited to, hydrogenated polydecene, squalane, and petrolatum.

From the viewpoint of further exerting the effects of the present invention, as the non-volatile oil, a non-volatile oil having a viscosity, at 25°C, of 100 mPa·s or less is preferable, and for example, a non-volatile oil having a viscosity, at 25 °C, of 100 mPa·s or less, 90 mPa·s or less, 80 mPa·s or less, 70 mPa·s or less, 60 mPa·s or less, 50 mPa·s or less, 40 mPa·s or less, 30 mPa·s or less, 20 mPa·s or less, or 10 mPa·s or less is preferable. The viscosity at 25°C may be 2.0 mPa.s or more or 5.0 mPa.s or more.

In the present invention, the viscosity can be confirmed by measurement with, for example, a BL type viscosity meter (manufactured by Shibaura System Co., Ltd.), rotor No. 3 at 12 rpm, for 60 seconds, at 25°C.

A commercially available product can be used as the non-volatile oil. Examples of commercially available non-volatile hydrocarbon oils may include, but are not limited to, hydrogenated polydecene, squalane, liquid paraffin, and petrolatum.

In the cosmetic of the present invention, from the viewpoint of suppressing the appearance of whiteness of the hydrophobized silica powder on the skin, the content of non-volatile oil, relative to the total amount of the cosmetic, may be 1.0 mass% or more, 2.0 mass% or more, 3.0 mass% or more, 4.0 mass% or more, 5.0 mass% or more, 6.0 mass% or more, 7.0 mass% or more, 8.0 mass% or more, 9.0 mass% or more, 10 mass% or more, 11 mass% or more, 12 mass% or more, 13 mass% or more, 14 mass% or more, 15 mass% or more, 16 mass% or more, 17 mass% or more, 18 mass% or more, 19 mass% or more, or 20 mass% or more, and from the viewpoint of improving sebum absorption, may be 40 mass% or less, 35 mass% or less, 30 mass% or less, 25 mass% or less, or 20 mass% or less relative to the total amount of the cosmetic.

From the viewpoint of considering the balance between the aim of suppressing whitening of the hydrophobized silica powder on the skin and the aim of improving the sebum absorption, the volume ratio of non-volatile oil to the maximum oil absorption amount of hydrophobized silica powder may be appropriately adjusted. For example, in the cosmetic of the present invention, the ratio of the volumetric content of the non-volatile oil to the maximum oil absorption amount of the hydrophobic silica powder ("volumetric content of non-volatile oil" / "maximum oil absorption amount of hydrophobic silica powder") may be, for example, 0.05 or more, 0.08 or more, 0.10 or more, 0.15 or more, 0.20 or more, 0.25 or more, 0.30 or more, 0.35 or more, 0.40 or more, 0.45 or more, 0.50 or more, 0.55 or more, 0.60 or more, 0.65 or more, 0.70 or more, or 0.75 or more, and may be less than 1.0, 0.95 or less, or 0.90 or less. The maximum oil absorption amount of the hydrophobic silica powder is a value obtained by multiplying the oil absorption amount (ml/g) of the hydrophobic silica powder for oleic acid by the content (g) of the hydrophobic silica powder contained in the cosmetic.

### <Dispersant>

The cosmetic of the present invention comprises a dispersant. The dispersant is primarily used to disperse the hydrophobized silica powder in the aqueous solvent.

In the present invention, the dispersant may be, for example, a polymer dispersant, and more specifically, an acrylic polymer or a crosslinked acrylic acid copolymer, but is not limited thereto.

Examples of the acrylic polymers include n-butyl (meth)acrylate, isobutyl (meth)acrylate, t-butyl (meth)acrylate, 2-ethylhexyl (meth)acrylate, n-hexyl (meth)acrylate, n-octyl (meth)acrylate, cyclohexyl (meth)acrylate, decyl (meth)acrylate, undecyl (meth)acrylate, lauryl (meth)acrylate, tridecyl (meth)acrylate, myristyl (meth)acrylate, pentadecyl (meth)acrylate, palmityl (meth)acrylate, heptadecyl (meth)acrylate, stearyl (meth)acrylate, isostearyl (meth)acrylate, oleyl (meth)acrylate, behenyl (meth)acrylate, (meth)acrylic acid ester having a linear, branched, or alicyclic hydrocarbon group; acrylonitrile; (meth)acrylamides such as acrylamide, diacetoneacrylamide, N,N-dimethylacrylamide, N-t-butylacrylamide, N-octylacrylamide, and N-t-octylacrylamide; sulfonic acid group-containing (meth)acrylamides such as 2-(meth)acrylamide-2-methylpropanesulfonic acid; alkylaminoalkyl (meth)acrylates such as aminoethyl (meth)acrylate, t-butylaminoethyl methacrylate, and methylaminoethyl (meth)acrylate; dialkylaminoalkyl (meth)acrylates such as dimethylaminoethyl (meth)acrylate and diethylaminoethyl (meth)acrylate; dialkylaminoalkyl (meth)methacrylamides such as dimethylaminoethyl (meth)acrylamide and diethylaminoethyl (meth)acrylamide; esters of a cyclic compound and meth)acrylic acid, such as tetrahydrofurfuryl (meth)acrylate, isobornyl (meth)acrylate, and glycidyl (meth)acrylate; (meth)acrylic acid alkoxyalkyl esters such as ethoxyethyl (meth)acrylate and methoxyethyl (meth)acrylate; monoesters of a polyalkylene glycol and (meth)acrylic acid such as polyethylene glycol mono(meth)acrylate and polypropylene glycol mono(meth)acrylate; sulfonic acid group-containing (meth)acrylic esters; methacryloyloxyalkyl phosphate monoesters such as (meth)acryloyloxyethyl phosphate; glyceryl (meth)acrylate, 2-methacryloyloxyethyl succinate, 2-(meth)acryloyloxyethyl phthalate, β-carboxyethyl acrylate, acryloyloxyethyl succinate, 2-(meth)acryloyloxyethyl tetrahydrophthalate, 2-(meth)acryloyloxyethyl hexahydrophthalate; 1,4-butanediol di(meth)acrylate, 1,6-hexanediol di(meth)acrylate, ethylene glycol di(meth)acrylate, polyethylene (n = 2 to 50) glycol di(meth)acrylate, propylene glycol di(meth)acrylate, polypropylene (n = 2 to 50) glycol di(meth)acrylate, butylene glycol di(meth)acrylate, dipentyl glycol di(meth)acrylate, neopentyl glycol di(meth)acrylate, trimethylolpropane di(meth)acrylate, methylenebisacrylamide, bisphenol F EO-modified (n = 2 to 50) di(meth)acrylate, bisphenol A EO-modified (n = 2 to 50) di(meth)acrylate, bisphenol S EO-modified (n = 2 to 50) di(meth)acrylate, trimethylolethane tri(meth)acrylate, trimethylolpropane tri(meth)acrylate, trimethylolpropane tricaprolactonate tri(meth)acrylate, trimethylolhexane tri(meth)acrylate, pentaerythritol tri(meth)acrylate, pentaerythritol tetra(meth)acrylate, diglycerin tetra(meth)acrylate, ditrimethylolpropane tetra(meth)acrylate, ditrimethylolpropane tetracaprolactonate tetra(meth)acrylate, ditrimethylolethane tetra(meth)acrylate, ditrimethylolbutane tetra(meth)acrylate, ditrimethylolhexane tetra(meth)acrylate, dipentaerythritol penta(meth)acrylate, dipentaerythritol hexa(meth)acrylate, tripentaerythritol hexa(meth)acrylate, and tripentaerythritol hepta(meth)acrylate.

Examples of crosslinked acrylic acid copolymers include, but are not limited to, acrylic acid-alkyl methacrylate copolymers, and more specifically, (acrylates/C₁₀₋₃₀ alkyl acrylate) crosspolymers and (hydroxyethyl acrylate/Na acryloyldimethyltaurate) copolymers.

Note that the dispersant may include a surfactant having a dispersing effect. The surfactant having a disperse effect may be selected from, for example, anionic surfactants, cationic surfactants, amphoteric surfactants, and nonionic surfactants.

In the cosmetic of the present invention, one or more dispersants can be used. The content of the dispersant in the cosmetic of the present invention may be, relative to the total amount of the cosmetic, 0.01 mass% or more, 0.03 mass% or more, 0.05 mass% or more, 0.1 mass% or more, 0.5 mass% or more, or 0.7 mass% or more, and may be 2.0 mass% or less, 1.5 mass% or less, or 1.0 mass% or less.

### <Aqueous Solvent>

The cosmetic of the present invention comprises an aqueous solvent. The aqueous solvent is a medium containing water as a primary component, and may be water alone, or may contain a lower alcohol and/or a polyhydric alcohol.

The water may be ion-exchanged water, purified water, or tap water.

Examples of lower alcohols include, but are not limited to, ethanol, propanol, and isopropanol.

Examples of polyhydric alcohols include, but are not limited to, dihydric alcohols such as ethylene glycol, propylene glycol, trimethylene glycol, 1,2-butylene glycol, 1,3-butylene glycol, tetramethylene glycol and 2,3-butylene glycol, or trihydric alcohols such as glycerin. Note that the polyhydric alcohols described above may be blended as other components (for example, moisturizing agent) contained in the cosmetic.

The content of the aqueous solvent in the cosmetic of the present invention may be, relative to the total amount of the cosmetic, 10 mass% or more, 20 mass% or more, 30 mass% or more, 40 mass% or more, or 50 mass% or more, and may be 95 mass% or less, 90 mass% or less, 85 mass% or less, or 80 mass% or less.

### <Other Components>

The cosmetic of the present invention may contain a total of 50 mass% or more, 60 mass% or more, 70 mass% or more, 80 mass% or more, or 90 mass% or more of the above components (i.e., the hydrophobized silica powder, non-volatile oil, dispersant, and aqueous solvent) relative to the total amount of the cosmetic. In addition to these components, the cosmetic of the present invention may further contain other components as long as they do not impair the effects of the present invention. Other components are exemplified below, but are not intended to limit the cosmetic of the present invention.

The cosmetic of the present invention may further contain a moisturizer. Examples of moisturizers include, but are not limited to, polyethylene glycol, propylene glycol, glycerin, 1,3-butylene glycol, xylitol, sorbitol, maltitol, chondroitin sulfate, hyaluronic acid, mucoitin sulfate, caronin acid, atelocollagen, sodium lactate, dl-pyrrolidone carboxylate, short-chain soluble collagen, diglycerin (EO) PO adduct, rose extract, yarrow extract, and melilot extract.

The cosmetic of the present invention may further contain powder components other than the hydrophobized silica powder. Examples of such powder components include, but are not limited to, inorganic powders (for example, talc, kaolin, mica, sericite, muscovite, phlogopite, synthetic mica, red mica, biotite, permiculite, magnesium carbonate, calcium carbonate, aluminum silicate, barium silicate, calcium silicate, magnesium silicate, strontium silicate, metal tungstate, magnesium, silica, zeolite, barium sulfate, calcined calcium sulfate (calcined gypsum), calcium phosphate, fluoroapatite, hydroxyapatite, ceramic powder, metal soaps (for example, zinc myristate, calcium palmitate, aluminum stearate), and boron nitride); organic powders (for example, polyamide resin powder (nylon powder), polyethylene powder, polymethyl methacrylate powder, polystyrene powder, styrene-acrylic acid copolymer resin powder, benzoguanamine resin powder, polytetrafluoroethylene powder, and cellulose powder); inorganic white pigments (for example, and zinc oxide); inorganic red pigments (for example, iron titanate); inorganic purple pigments (for example, mango violet, and cobalt violet); inorganic green pigments (for example, chromium oxide, chromium hydroxide, and cobalt titanate); inorganic blue pigments (for example, ultramarine blue, and dark blue); pearl pigments (for example, titanium oxide-coated mica, titanium oxide-*coated bismuth oxychloride, titanium oxide-coated talc, colored titanium oxide-coated mica, bismuth oxychloride, and fish scale foil); metal powder pigments (for example, aluminum powder and copper powder); organic pigments such as zirconium, barium, or aluminum lake dyes (for example, organic pigments such as red no. 201, red no. 202, red no. 204, red no. 205, red no. 220, red no. 226, red no. 228, red no. 405, orange no. 203, orange no. 204, yellow no. 205, yellow no. 401, and blue no. 404, as well as red no. 3, red no. 104, red no. 106, red no. 227, red no. 230, red no. 401, red no. 505, orange no. 205, yellow no. 4, yellow no. 5, yellow no. 202, yellow no. 203, green no. 3, and blue no. 1); and natural pigments (for example, chlorophyll and β-carotene).

The cosmetic of the present invention may further comprise a liquid fat. Examples of liquid fats include, but are not limited to, avocado oil, camellia oil, macadamia nut oil, corn oil, olive oil, rapeseed oil, sesame oil, persic oil, wheat germ oil, sasanqua oil, castor oil, linseed oil, safflower oil, cottonseed oil, perilla oil, soybean oil, peanut oil, teaseed oil, kaya oil, rice bran oil, sinagiri oil, Japanese paulownia oil, jojoba oil, germ oil, and triglycerin.

The cosmetic of the present invention may further comprise a solid fat. Examples of solid fats include, but are not limited to, cacao butter, coconut oil, hydrogenated coconut oil, palm oil, palm kernel oil, Japanese magnolia kernel oil, hydrogenated oil, Japanese magnolia, and hydrogenated castor oil.

The cosmetic of the present invention may further comprise a wax. Examples of waxes include, but are not limited to, beeswax, candelilla wax, cotton wax, carnauba wax, bayberry wax, privet wax, montan wax, bran wax, kapok wax, sugarcane wax, hexyl laurate, jojoba wax, shellac wax, POE lanolin alcohol ether, POE lanolin alcohol acetate, and POE cholesterol ether.

The cosmetic of the present invention may further comprise a higher fatty acid. Examples of higher fatty acids include, but are not limited to, lauric acid, myristic acid, palmitic acid, stearic acid, behenic acid, oleic acid, undecylenic acid, thoric acid, isostearic acid, linoleic acid, linolic acid, eicosapentaenoic acid (EPA), and docosahexaenoic acid (DHA).

The cosmetic of the present invention may further comprise a higher alcohol. Examples of higher alcohols include, but are not limited to, straight-chain alcohols (for example, lauryl alcohol, cetyl alcohol, stearyl alcohol, behenyl alcohol, myristyl alcohol, oleyl alcohol, and cetostearyl alcohol); and branched-chain alcohols (for example, monostearyl glycerin ether (bacyl alcohol), 2-decyltetradecinol, cholesterol, phytosterol, hexyldodecanol, isostearyl alcohol, and octyldodecanol).

The cosmetic of the present invention may further comprise, in addition to the dispersant, a surfactant for the purpose of emulsification. Such surfactant may be selected from, for example, anionic surfactants, cationic surfactants, amphoteric surfactants, and nonionic surfactants.

The cosmetic of the present invention may further comprise a UV absorber. Examples of UV absorbers include, but are not limited to, benzoic acid-based UV absorbers (for example, para-aminobenzoic acid (hereinafter abbreviated as PABA), PABA monoglycerin ester, N,N-dipropoxy PABA ethyl ester, N,N-diethoxy PABA ethyl ester, N,N-dimethyl PABA ethyl ester, N,N-dimethyl PABA butyl ester, and N,N-dimethyl PABA ethyl ester); anthranilic acid-based UV absorbers (for example, homomenthyl-N-acetylanthranilate); salicylic acid-based UV absorbers (for example, amyl salicylate, menthyl salicylate, homomenthyl salicylate, octyl salicylate, phenyl salicylate, benzyl salicylate, and p-isopropanol phenyl salicylate); cinnamic acid-based UV absorbers (for example, octyl cinnamate, ethyl-4-isopropyl cinnamate, methyl-2,5-diisopropyl cinnamate, ethyl-2,4-diisopropyl cinnamate, methyl-2,4-diisopropylcinnamate, propyl-p-methoxycinnamate, isopropyl-p-methoxycinnamate, isoamyl-p-methoxycinnamate, octyl-p-methoxycinnamate (2-ethylhexyl-p-methoxycinnamate), 2-ethoxyethyl-p-methoxycinnamate, cyclohexyl-p-methoxycinnamate, ethyl-α-cyano-β-phenylcinnamate, 2-ethylhexyl-α-cyano-β-phenylcinnamate, and glyceryl mono-2-ethylhexanoyl-di-para-methoxycinnamate); benzophenone-based UV absorbers (for example, 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone, and 4-hydroxy-3-carboxybenzophenone); 3-(4'-methylbenzylidene)-d,l-camphor, 3-benzylidene-d,l-camphor; 2-phenyl-5-methylbenzoxazole; 2,2'-hydroxy-5-methylphenylbenzotriazole ; 2-(2'-hydroxy-5'-t-octylphenyl)benzotriazole; 2-(2'-hydroxy-5'-methylphenylbenzotriazole; dibenzalazine; dianisoylmethane; 4-methoxy-4'-t-butyldibenzoylmethane; and 5-(3,3-dimethyl-2-norbomylidene)-3-pentan-2-one.

Examples of other components which can be blended in the cosmetic of the present invention include, but are not limited to, water-soluble polymers, thickeners, monosaccharides, oligosaccharides, organic amines, alkylene oxide derivatives, sequestering agents, antioxidant aids, preservatives, whitening agents, blood circulation promoters, various extracts, activators, and antiseborrheic agents.

### <Viscosity of Cosmetic>

The viscosity of the cosmetic of the present invention is preferably 100000 mPa·s or less at 25°C, and more specifically, may be, for example, 100000 mPa·s or less, 80000 mPa·s or less, 50000 mPa·s or less, 10000 mPa·s or less, 8000 mPa·s or less, 5000 mPa·s or less, or 1000 mPa·s or less. When the viscosity is 100000 mPa·s or less, a skin care sensation of the cosmetic can be obtained. Furthermore, from the viewpoint of suppressing sedimentation of the hydrophobized silica powder, the viscosity of the cosmetic is preferably 100 mPa·s or more.

### <Use of Cosmetic>

The cosmetic of the present invention may be, for example, for use in pore correction and/or shine suppression. As used herein, "pore correction" means to make unevenness of pores inconspicuous. The term "shine suppression" refers to suppression of shine due to sebum by absorption of sebum into the pores.

The form of the cosmetic of the present invention is arbitrary, and may be, for example, a solution system, emulsification system, lotion, gel, mist, spray, or mousse.

The product form of the cosmetic of the present invention is not particularly limited, and it can be suitably used for, for example, skin care cosmetics such as lotions, milky lotions, serums, creams, whitening agents, eye care creams and sunscreen cosmetics, and makeup cosmetics such as foundations and makeup bases.

### <<Method for Production of Cosmetic>>

The cosmetic of the present invention can be produced by, for example, adding the hydrophobized silica powder, dispersant, and other optional components to the aqueous solvent, dispersing the hydrophobized silica powder, and thereafter adding a non-volatile appropriately selected in accordance with the type of the hydrophobized silica powder.

### EXAMPLES

The present invention will be described in more detail below with reference to the Examples, but the present invention is not limited to these. Note that below, unless otherwise specified, the contents are shown in mass%.

### <Preparation of Cosmetic Sample of Each of Examples and Comparative Examples>

In accordance with the formulations shown in Tables 1 to 3, a hydrophobized silica powder, a dispersant, and other components were added to an aqueous solvent, and the hydrophobized silica powder was dispersed, and an oil was added thereto to prepare each cosmetic sample.

### <Evaluation>

### (Evaluation of Whiteness)

Each of the cosmetic samples prepared above was applied to a black *Suprare* (artificial leather), and after 15 minutes, it was evaluated by five expert panelists based on the following criteria. Note that the results are shown in Table 1.

### Evaluation Criteria:

"A": three or more out of the five panelists answered that whiteness was not noticeable;
"B": three or more out of the five panelists answered that whiteness was not noticeable or that the whiteness was noticeable but only barely (excluding the case where three or more out of the five panelists answered that whiteness was not noticeable);
"C": three or more out of the five panelists answered that whiteness was noticeable.

### (Evaluation of Sebum Absorption Effect)

25 µl of each cosmetic sample prepared above was applied to a 5 cm × 5 cm artificial leather ("*Suprare*", manufactured by Ideatex Japan Co., Ltd.) and allowed to stand for 15 minutes. Thereafter, 15 µl of oleic acid was further applied thereon and allowed to stand for 30 minutes. Using a gloss meter "VG-2000" (manufactured by Nippon Denshoku Industries Co., Ltd.), the gloss value was measured three times for each sample on the black *Suprare*, and the average obtained gloss values are shown in Table 4.

It should be noted that the lower the gloss value, the greater shine is suppressed, i.e., the higher the sebum absorption effect. When the gloss value is 3 or less, it is judged that there is a sebum absorption effect. Furthermore, in Tables 1 to 3, the sebum absorption effects of the measured Examples were evaluated based on the following criteria.

"A": the gloss value is less than 2;
"B": the gloss value is 2 or more and 3 or less.

**[Table 1]**

| (Table 1: Examples 1 to 11) | | | | | | | | | | | |
|---|---|---|---|---|---|---|---|---|---|---|---|
| Components | Example 1 | Example 2 | Example 3 | Example 4 | Example 5 | Example 6 | Example 7 | Example 8 | Example 9 | Example 10 | Example 11 |
| Deionized water | Remainder | Remainder | Remainder | Remainder | Remainder | Remainder | Remainder | Remainder | Remainder | Remainder | Remainder |
| Dimethicone-treated silica (Particle size: 5.0 µm) (Oil absorption amt ¹⁾: 2.34 ml/g) | 10 | 10 | 10 | 10 | 10 | 10 | 10 | 10 | 10 | 10 | - |
| Stearic acid-treated silica (Particle size: 4.8 µm) (Oil absorption amt: 1.96 ml/g) | - | - | - | - | - | - | - | - | - | - | 10 |
| Non-volatile dimethicone (Viscosity: 6 mPa·s) (Density: 0.92 g/ml) | 2.0 | 4.0 | 6.0 | 8.0 | 10 | 12 | 14 | 16 | 18 | - | - |
| Non-volatile dimethicone (Viscosity: 20 mPa·s) (Density: 0.95 g/ml) | - | - | - | - | - | - | - | - | - | 4.0 | - |
| Hydrogenated polydecene (non-volatized) (Viscosity: 30 mPa·s) (Density: 0.82 g/ml) | - | - | - | - | - | - | - | - | - | - | 4.0 |
| Other (common) components | 19 | 19 | 19 | 19 | 19 | 19 | 19 | 19 | 19 | 19 | 19 |
| Total (mass%) | 100 | 100 | 100 | 100 | 100 | 100 | 100 | 100 | 100 | 100 | 100 |
| "Volumetric content of non-volatile oil" / "Maximum oil absorption amount of hydrophobic silica powder" | 0.093 | 0.186 | 0.278 | 0.371 | 0.464 | 0.558 | 0.650 | 0.743 | 0.836 | 0.180 | 0.208 |
| Whiteness evaluation | B | B | B | A | A | A | A | A | A | B | A |
| Sebum absorption effect evaluation | A | A | A | n.d. | A | n.d. | n.d. | A | A | B | B |

| | | | | | | | | | | | |
|---|---|---|---|---|---|---|---|---|---|---|---|
| Note: 1) The "oil absorption amt" of the silica powder is the oil absorption amount for the oleic acid; the same applies in the Comparative Examples. | | | | | | | | | | | |

**[Table 2]**

| (Table 2: Comparative Examples 1 to 12) | | | | | | | | | | | | |
|---|---|---|---|---|---|---|---|---|---|---|---|---|
| Components | Comparative Example 1 | Comparative Example 2 | Comparative Example 3 | Comparative Example 4 | Comparative Example 5 | Comparative Example 6 | Comparative Example 7 | Comparative Example 8 | Comparative Example 9 | Comparative Example 10 | Comparative Example 11 | Comparative Example 12 |
| Deionized water | Remainder | Remainder | Remainder | Remainder | Remainder | Remainder | Remainder | Remainder | Remainder | Remainder | Remainder | Remainder |
| Amodimethicone-treated silica (Particle size: 4.8 µm) (Oil absorption amt: 1.69 ml/g) | 10 | - | - | 14.81 | - | - | - | - | - | - | - | - |
| Triethoxy caprylyl silane-treated silica (Particle size: 9.0 µm) (Oil absorption amt: 1.43 ml/g) | - | 10 | - | - | - | - | - | - | - | - | - | - |
| Trimethylsilylated silica (Particle size: 10 µm) (Oil absorption amt: 8.46 ml/g) | - | - | 6.0 | - | 2.96 | - | - | - | - | - | - | - |
| Dimethicone-treated silica (Particle size: 5.0 µm) (Oil absorption amt: 2.34 ml/g) | - | - | - | - | - | 10 | - | 10 | 10 | 10 | 10 | 10 |
| Hydrogen dimethicone-treated silica (Particle size: 5.0 µm) (Oil absorption amt: 2.13 ml/g) | - | - | - | - | - | - | 10 | - | - | - | - | - |
| Triethylhexanoin (Viscosity: 25 mPa·s) | 8.0 | 8.0 | 8.0 | 8.0 | 8.0 | 8.0 | 8.0 | 0.0 | 1.0 | 2.0 | 4.0 | 6.0 |
| Other (common) components | 19 | 19 | 19 | 19 | 19 | 19 | 19 | 19 | 19 | 19 | 19 | 19 |
| Total (mass%) | 100 | 100 | 100 | 100 | 100 | 100 | 100 | 100 | 100 | 100 | 100 | 100 |
| Whiteness evaluation | C | C | C | C | C | C | C | C | C | C | C | C |
| Sebum absorption effect evaluation | n.d. | n.d. | n.d. | n.d. | n.d. | n.d. | n.d. | n.d. | n.d. | n.d. | n.d. | n.d. |

**[Table 3]**

| (Table 3: Comparative Examples 13 to 25) | | | | | | | | | | | | | |
|---|---|---|---|---|---|---|---|---|---|---|---|---|---|
| Components | Comparative Example 13 | Comparative Example 14 | Comparative Example 15 | Comparative Example 16 | Comparative Example 17 | Comparative Example 18 | Comparative Example 19 | Comparative Example 20 | Comparative Example 21 | Comparative Example 22 | Comparative Example 23 | Comparative Example 24 | Comparative Example 25 |
| Deionized water | Remainder | Remainder | Remainder | Remainder | Remainder | Remainder | Remainder | Remainder | Remainder | Remainder | Remainder | Remainder | Remainder |
| Dimethicone-treated silica (Particle size: 5.0 µm) (Oil absorption amt: 2.34 ml/g) | 10 | 10 | 10 | 10 | 10 | 10 | 10 | 10 | 10 | 10 | 10 | 10 | 10 |
| Diphenylsiloxyphenyl trimethicone (Viscosity: 50 mPa·s) | 4.0 | - | - | - | - | - | - | - | - | - | - | - | - |
| Diisopropyl sebacate | - | 4.0 | 6.0 | 8.0 | 10 | - | - | - | - | - | - | - | - |
| Mineral oil (Viscosity: 23 mPa·s) | - | - | - | - | - | 4.0 | - | - | - | - | - | - | - |
| Caprylyl methicone (Viscosity: 3 mPa·s) | - | - | - | - | - | - | 4.0 | - | - | - | - | - | - |
| Isopropyl myristate (Viscosity: 5 mPa.s) | - | - | - | - | - | - | - | 4.0 | 6.0 | - | - | - | - |
| (C₁₂₋₁₅) alkyl benzoate | - | - | - | - | - | - | - | - | - | 4.0 | 6.0 | - | - |
| Squalane | - | - | - | - | - | - | - | - | - | - | - | 4.0 | 3.0 |
| Other (common) components | 19 | 19 | 19 | 19 | 19 | 19 | 19 | 19 | 19 | 19 | 19 | 19 | 19 |
| Total (mass%) | 100 | 100 | 100 | 100 | 100 | 100 | 100 | 100 | 100 | 100 | 100 | 100 | 100 |
| Whiteness evaluation | C | C | C | C | C | C | C | C | C | C | C | C | C |
| Sebum absorption effect evaluation | n.d. | n.d. | n.d. | n.d. | n.d. | n.d. | n.d. | n.d. | n.d. | n.d. | n.d. | n.d. | n.d. |

The other (common) components in Tables 1 and 2 are as follows:
Other components - content (mass%)
Sodium metaphosphate - appropriate amount
Glycerin - 9.0
Dipropylene glycol - 1.0
1.3-Butylene glycol - 8.0
Phenoxyethanol - 0.5
(Acrylates/C10-30 alkyl acrylate) crosspolymer - 0.03
Potassium hydroxide - appropriate amount
(Hydroxyethyl acrylate/Na acryloyldimethyltaurate) copolymer - 0.75
Total (mass% of total amount of cosmetic) - 19

**[Table 4]**

| | Before application of oleic acid (5 minutes after application of base) (Average value of 3 measurements) | 30 minutes after application of oleic acid (Average value of 3 measurements) |
|---|---|---|
| No coating | 1.3 | 4.80 |
| Example 1 | 1.2 | 1.23 |
| Example 2 | 1.1 | 1.17 |
| Example 3 | 1.1 | 1.47 |
| Example 5 | 1.1 | 1.43 |
| Example 8 | 1.1 | 1.43 |
| Example 9 | 1.1 | 1.57 |
| Example 10 | 0.7 | 2.00 |
| Example 11 | 1.1 | 2.43 |

As is clear from Tables 1 to 4, all of the cosmetics of Examples 1 to 11 did not whiten on the skin after application, and were capable of absorbing sebum.

## Claims

1. A cosmetic, comprising:
an aqueous solvent,
a hydrophobized silica powder dispersed in the aqueous solvent,
a non-volatile oil, and
a dispersant, wherein
(i) the hydrophobized silica powder has been subjected to a hydrophobic treatment with a silicone-based hydrophobizing agent and the non-volatile oil is a non-volatile silicone oil, or
(ii) the hydrophobized silica powder has been subjected to a hydrophobic treatment with a fatty acid-based hydrophobizing agent and the non-volatile oil is a non-volatile hydrocarbon oil.

2. The cosmetic according to claim 1, wherein the hydrophobized silica powder has been subjected to a hydrophobic treatment with a dimethicone-based hydrophobizing agent and the non-volatile oil is a non-volatile dimethicone.

3. The cosmetic according to claim 1 or 2, wherein an oleic acid oil absorption amount of the hydrophobized silica powder is 1.0 ml/g or more.

4. The cosmetic according to any one of claims 1 to 3, wherein a content of the hydrophobized silica powder is 1.0 to 20 mass%.

5. The cosmetic according to any one of claims 1 to 4, wherein a content of the non-volatile oil is 1.0 to 40 mass%.

6. The cosmetic according to any one of claims 1 to 5, wherein the cosmetic is for use inpore correction.

7. The cosmetic according to any one of claims 1 to 6, wherein a viscosity of the cosmetic is 100000 mPa.s or less at 25°C.

8. The cosmetic according to any one of claims 1 to 7, wherein a ratio of a volumetric content of the non-volatile oil to a maximum oil absorption amount of the hydrophobic silica powder ("volumetric content of non-volatile oil" / "maximum oil absorption amount of hydrophobic silica powder") is 0.3 or greater and less than 1.0.
